# EUROPEAN PATENT APPLICATION

(11) **EP 1 380 829 A1**
(43) Date of publication of application: **14.01.2004**
(21) Application number: 01921958.3
(22) Date of filing: 20.04.2001
(51) Int. Cl.: G01N 1/10

(54) **INSTRUMENT FOR USE IN COLLECTING AND RECOVERING LIQUID SECRETION IN ORAL CAVITY**

(71) Applicant: Sapporo Immuno Diagnostic Laboratory, Sapporo-shi, Hokkaido 001-0922 (JP)
(72) Inventor: YOKOYAMA, Toru, Sapporo Immuno Diagnostic Lab., Sapporo-shi, Hokkaido 001-0922 (JP); SHINOZUKA, Naoki, Sapporo Immuno Diagnostic Lab., Sapporo-shi, Hokkaido 001-0922 (JP); NAKAMURA, Kenji, Sapporo Immuno Diagnostic Lab., Sapporo-shi, Hokkaido 001-0922 (JP)
(74) Representative: HOFFMANN - EITLE
(86) International application number: PCT/JP2001/003408
(87) International publication number: WO 2002/086453

(57) **Abstract**

The present invention provides a sampling/recovery instrument characterized by an oral secreta liquid sampling method which samples an oral secreta liquid either simultaneously with sterilizing it, or after sterilizing oral bacteria with a bactericide, to suppress the decomposition of D-glucose by bacteria, in order to determine D-glucose in the oral secreta liquid. Concretely, the invention provides an oral secreta liquid sampling/recovery laminated simplified instrument produced by a very easy manufacturing method using inexpensive members, the instrument being characterized by sterilely and antibacterially sampling an oral secreta liquid with the use of a hydrophilic absorbent containing a bactericidal ingredient and/or an antibacterial ingredient, and pressurizing the hydrophilic absorbent toward a recovery hole to recover the absorbed oral secreta liquid. By this means, the invention realizes the rapid sterilization; sampling and recovery of samples.

## Description

### FIELD OF THE INVENTION

This invention relates to a disposable simplified instrument aimed to determine D-glucose in samples, such as oral secreta liquids, for example, saliva from the salivary glands, including the parotid, submandibular and sublingual glands, and exudate from the gingival sulcus. This instrument can not only sterilize bacteria existent in the oral cavity, but also sample an oral secreta liquid, and even recover the liquid. The instrument can be produced by a simple manufacturing method using inexpensive members.

### BACKGROUND OF THE INVENTION

Currently, diabetes is increasing in incidence on a global scale, and the achievement of a noninvasive testing method is eagerly desired for patients with diabetes in whom frequent blood sampling is inevitable for good sugar monitoring.

The inventors of the present invention, have proceeded with studies of methods and instruments for sampling of oral secreta liquids for determination of D-glucose in the saliva as noninvasive means (e.g. WO02/31105). Through these studies, we have shown the usefulness of saliva as a diagnostic material. In these studies, we have sterilized bacteria present in the oral cavity, thereby elucidating the effect of the sterilization in suppressing the decomposition of D-glucose by bacteria.

Examinations using saliva as a sample (Annals of the New York Academy of Sciences, Vol. 694, p. 216(1993)) include various screening tests for HIV, hepatitis virus, syphilis, rubella and measles, and pharmaceutical and drug monitoring tests for therapeutics such as theophylline and phenytoin, and pharmaceuticals and drugs such as nicotine and cocaine (Japanese Journal of Clinical Laboratory Automation, Vol. 19, p. 265(1994)) using a saliva sampling instrument "OraSure" (Epitope, Inc.) (e.g. WO97/24979 (1997), WO96/04850 (1996), WO95/27205 (1995)), a saliva sampling instrument "Omnisal (Saliva-Sampler)" (Saliva Diagnostic Systems, Inc.) (e.g. WO95/02996 (1995), U.S. Patent No. 5,260,031 (1993)), and a saliva sampling instrument "Salivette" (Sarstedt) (e.g. U.S. Patent No. 4,774,962 (1988)).

The above-mentioned instruments are used for tasks ranging from sampling to preservation, and are composed of two dedicated instruments including a sampling instrument and a preservation tube containing a preservative liquid. In terms of form, the sampling instrument consists of an absorption pad and a holder (the saliva sampling instruments "OraSure" and "Omnisal(Saliva·Sampler)"). These constituent parts are not fixed firmly, and the absorption pad is likely to be detached in the oral cavity. Moreover, the holder has a possibility for damaging the interior of the oral cavity because the holder has little flexibility, and the conditions for its disposition in the oral cavity are limited.

Sodium azide is incorporated into the preservative liquid in order to prevent proliferation of oral bacteria present in the samples taken (the saliva sampling instrument "Omnisal(Saliva·Sampler)"). However, the test items do not include the determination of D-glucose, and no sterilization procedure is performed during sampling. Nor is the test on the spot, so-called POCT (point-of-care testing), conducted. The sample accommodated in a preservation container is transported to a clinical laboratory or the like, only where a recovery operation for the sample is carried out, and a test is conducted.

A sampling/measuring instrument involving two steps until measurement ("OraQuick" (Epitope, Inc.); e.g. WO99/50656 (1999)) is an HIV test kit by lateral flow immunochromatography with an extended absorption pad. This is a POCT article which obtains test results in about 20 minutes.

This instrument is of a measuring unit integrated type, and can omit a recovery step, but a reaction reagent is also included therein integrally. Possibilities for reverse inflow of the reagent into the oral cavity, and accidental swallowing of the reagent are also raised. With the instrument, the determination of D-glucose is not performed, and no sterilization procedure is performed during sampling.

As an integral method, a sampling unit integrated sensor or plate (e.g. JP 2000-9728) is named. For this sensor or plate, risks of reverse inflow and accidental swallowing of the reagent, various modes of sampling, and rinsing of the salivary glands are described, but there is no description of sterilization.

Next, for intraoral cleaning using a cleaning fluid, there is a method of pre-sampling cleaning for determination of D-glucose (e.g. JP 9-72900). This method requires complicated removal treatment using a dedicated instrument, because a liquid is sprinkled as the cleaning fluid. Furthermore, this publication has no clear description of a method for sterilization.

Various washes containing bactericides have been developed and sold as liquid dentifrices (e.g. WO91/18585 (1991)). However, their purpose is to prevent dental caries. Moreover, they have to be used before sampling of an oral secreta liquid. They should be removed before this sampling, and the dilution of the oral secreta liquid is highly likely depending on the conditions of their removal.

In recent years, various types of POCT equipment have begun to be developed because of marked progresses in biosensor techniques, and there has been an increasing need for simplified instruments which are capable of recovery immediately after collection of samples.

Besides, the above instruments are put to disposable use in consideration of infection, etc. Thus, the instruments face major problems, such as compactness in size, and a decrease in the number of members used, thereby keeping the amount of waste to a minimum required level.

As discussed above, no proposals have been made for a method which applies treatment for sterilizing oral bacteria when sampling an oral secreta liquid in order to determine, at least, D-glucose in the oral secreta liquid. Nor have any proposals been made for a method which performs sampling simultaneously with sterilization of oral bacteria, or for a simplified instrument capable of recovering a sample after sampling as well as sterilization. Nor has any simplified sampling/recovery instrument for POCT use been shown at all.

### SUMMARY OF THE INVENTION

To solve the above-described problems, the present invention provides, first, a sampling/recovery instrument characterized by an oral secreta liquid sampling method which samples an oral secreta liquid either simultaneously with sterilizing the liquid, or after sterilizing oral bacteria with a bactericide to suppress the decomposition of D-glucose by bacteria, in order to determine D-glucose in the oral secreta liquid.

Next, the present invention provides an oral secreta liquid sampling/recovery laminated simplified instrument produced by a very easy manufacturing method using inexpensive members, the instrument being characterized by sterilely and antibacterially sampling an oral secreta liquid, without diluting the oral secreta liquid, with the use of a hydrophilic absorbent containing a bactericidal ingredient and/or an antibacterial ingredient, and pressurizing the hydrophilic absorbent toward a recovery hole to recover the absorbed oral secreta liquid. By this means, the invention realizes the rapid sterilization, sampling and recovery of a sample.

### DESCRIPTION OF THE INVENTION

Firstly, the present invention provides an oral secreta liquid sampling/recovery instrument characterized by a sampling method which samples an oral secreta liquid either simultaneously with sterilizing bacteria with a bactericidal ingredient, or after applying sterilizing treatment to the bacteria, the bacterial being present in various types and large amounts in the oral cavity and utilizing D-glucose as a nutrient source, when determining D-glucose contained in the oral secreta liquid.

As a second characteristic, the present invention provides an oral secreta liquid sampling/recovery laminated simplified instrument for POCT use, which uses inexpensive members, which has a small number of components, which is produced using an easy manufacturing method, and which is convenient to handle, the instrument being characterized by sterilely and antibacterially sampling an oral secreta liquid, without diluting the oral secreta liquid, with the use of a hydrophilic absorbent containing a bactericidal ingredient and/or an antibacterial ingredient, and pressurizing the hydrophilic absorbent to recover the absorbed oral secreta liquid.

The present invention also provides a sampling/recovery instrument which finds the bactericidal effect of a quaternary ammonium salt-based bactericidal ingredient, as a means of sterilization of oral bacteria, on *Streptococcus mutans* (a group of cariogenic bacteria which are present in dental plaque in the oral cavity and fermentatively decompose various monosaccharides to produce several types of organic acids) from the time course of D-glucose (e.g. W002/31105); and which adds an antibacterial ingredient in combination with the above bactericidal ingredient to show the antibacterial function of suppressing the growth of residual bacteria in addition to sterilization.

To promote salivation, the invention also provides a method which jointly uses a bactericide incorporating suitable additives not interfering with D-glucose determination, such as a flavor and an aromatic, in addition to the aforementioned bactericidal ingredient. In this case, the invention also provides a sampling/recovery instrument having the hydrophilic absorbent containing only the antibacterial ingredient and excluding the bactericidal ingredient, or a sampling/recovery instrument having the hydrophilic absorbent containing both the bactericidal ingredient and the antibacterial ingredient.

The oral secreta liquid sampling/recovery instrument of the present invention has a laminated structure composed of a hydrophilic absorbent, which is a flexible nonwoven fabric containing the aforementioned bactericidal ingredient and/or antibacterial ingredient, and a water resistant substrate, which is a transparent flexible film, the face and back of the hydrophilic absorbent being entirely covered with the water resistant substrate by thermocompression bonding, with a partial opening portion being retained. In the oral secreta liquid sampling/recovery instrument, an absorption portion, a liquid holding portion, a held liquid amount confirmation/display portion, and a recovery hole for the oral secreta liquid are arranged consecutively in a straight line.

The hydrophilic absorbent consists of a flexible nonwoven fabric, and has a structure and a nature by which to adsorb impurities contained in the oral secreta liquid, thus possessing a separation function capable of removing the impurities during recovery. Moreover, a multilayer configuration having a filtration layer, etc. of various pore sizes added to the absorbent makes it possible to separate the oral secreta liquid through the recovery hole after its passage through the filtration layer, the oral secreta liquid having a pore size not larger than the pore size selected for the filtration layer. The absorption status of the oral secreta liquid can be visually observed with ease by making the absorbent white, light-colored, e.g. milk-white, or translucent.

The water resistant substrate has a multilayer structure including a heat fusion layer in a transparent flexible film. Because of this structure, a convenient laminating/manufacturing process by thermocompression bonding can be achieved without particular need for a solvent or a pressure sensitive adhesive. Furthermore, thermocompression bonding, with the hydrophilic absorbent being disposed between the water resistant substrates, realizes an integral structure, thus preventing detachment of the members.

The absorption portion has openings of various shapes provided in the water resistant substrate to expose the hydrophilic absorbent located at the inner surface of the water resistant substrates.

The liquid holding portion is located at a position continuous with the absorption portion, and has a structure covered with the water resistant substrate for avoiding drying.

The held liquid amount confirmation/display portion is located above the liquid holding portion, and is characterized by a very simple and convenient mechanism such that the oral secreta liquid, absorbed from the absorption portion, wets the display portion to change optical transmission, whereby a colored portion on the back side of the hydrophilic absorbent in the display portion can be visually confirmed from the front.

A saliva sampling instrument "Omnisal (Saliva·Sampler)" is named as an instrument provided with an indicator of the sampling status. However, a reaction reagent is contained in an absorption pad for display by chemical color development, and may involve a risk of reversed inflow into the oral cavity or accidental swallowing.

The recovery hole is located on the end surface side of the liquid holding portion. The liquid holding portion, which has absorbed the oral secreta liquid and swollen, is pressurized toward the recovery hole, whereby the oral secreta liquid is recovered through the recovery hole. The liquid holding portion is covered, so that adhesion of a sample to the user can be avoided.

Since the hydrophilic absorbent and the water resistant substrate are flexible, as noted above, they can be easily disposed in a mazy region of the oral cavity, for example, near the parotid gland or below the tongue, when an oral secreta liquid is to be sampled. During recovery, moreover, the liquid holding portion is easy to pressurize, and spotting of the recovered sample to POCT equipment can also be performed with high efficiency.

A sensor chip and a sensor meter, which belong to POCT equipment, have already applied the principle of reaction and manufacturing techniques that we invented (Japanese Unexamined Patent Publication No. 2000-35413, WO00/04378, WO00/57166, WO02/18627, WO02/18924). The sensor chip consists of, at least, a working electrode and a counter electrode formed with the use of an electrically conductive material, and has a layered structure having a reaction reagent immobilized onto the upper surface of the electrode reaction portion thereof. The sensor meter consists of an electrochemical detector, etc.

As described above, the oral secreta liquid. sampling/recovery laminated simplified instrument containing a bactericidal ingredient according to the present invention has devised a very easy manufacturing method using inexpensive members, and has achieved convenient operability. Furthermore, steps ranging from sampling to recovery are realized by a one-part compact simplified instrument, and can be performed by a three-part configuration having, added to the one part, at most, a bactericide and a pressurizing implement for assisting in recovery. The decrease in the number of constituent parts, compactness, etc. have led to reduction in waste.

### BRIEF DESCRIPTION OF THE DRAWINGS

FIG. 1 shows, by an exploded view, an oral secreta liquid sampling/recovery instrument in Embodiment 1 of the present invention, and also shows, by a sectional view of a human oral cavity, an example of intraoral use of the instrument;
FIG. 2 shows a held liquid amount confirmation/display portion of the oral secreta liquid sampling/recovery instrument;
FIG. 3 shows a recovery hole of the oral secreta liquid sampling/recovery instrument; and
FIG. 4 shows the constituent elements of an oral secreta liquid sampling/recovery kit in Embodiment 2.

In the drawings, the reference numerals are described as follows:

1 denotes a water resistant substrate; 2, a hydrophilic absorbent; 3, an oral secreta liquid absorption portion; 4, a liquid holding portion; 5, a held liquid amount confirmation/display portion; 6, a recovery hole; 7, an oral secreta liquid sampling/recovery instrument; 8, an oral secreta liquid; 9, a bactericide; and 10, a pressurizing implement.

### DESCRIPTION OF THE PREFERRED EMBODIMENTS

A bactericidal ingredient, an antibacterial ingredient, and a bactericide, which are used in the present invention, are not limited, if they can be used in the oral cavity without affecting the human body, sterilize oral bacteria decomposing D-glucose, do not interfere with determination of D-glucose, are gaseous or solid in form, and do not dilute oral secreta liquids. Examples of the bactericidal ingredient are quaternary ammonium salts, such as cetylpyridinium chloride, benzalkonium chloride, and benzethonium chloride. Examples of the antibacterial ingredient are chitin-chitosan, silver, copper, and zinc. These ingredients can be used alone or in combination.

The bactericide, termed herein, refers to that which contains the above-mentioned bactericidal ingredient, and incorporates other suitable additives, such as a flavor, an aromatic, an excipient, a stabilizer, and a preservative.

The hydrophilic absorbent is not limited, if it can be used in the oral cavity without affecting the human body, if it can contain the bactericidal ingredient and the antibacterial ingredient, if it is free from nonspecific unintended adsorption of the sampled oral secreta liquid and free from contamination of the oral secreta liquid, if it is a hydrophilic material absorbing the oral secreta liquid, and if it has such mechanical strength as not to be damaged during use. For example, there can be used fibers such as cotton, glass fiber, silica fiber, and cellulose fiber, carboxymethylcellulose, diethylaminoethyl cellulose, cellulose acetate, cellulose mixed ester, nylon, nitrocellulose, polyether sulfone, polyester, polyethylene, polycarbonate, polytetrafluoroethylene, polyvinylidene fluoride, polyvinylidene difluoride, polypropylene, rayon, nonwoven fabric, and blends of the above fibers.

The water resistant substrate is not limited, if it can be used in the oral cavity without affecting the human body, if it can incorporate or can be coated with the bactericidal ingredient and the antibacterial ingredient, if it is free from nonspecific unintended adsorption of the sampled oral secreta liquid and free from contamination of the oral secreta liquid, if it is flexible and water resistant, if is highly workable, and if it has such mechanical strength as not to be damaged during use. For example, there can be used films of silicone, nylon, polyester, polyethylene, polyethylene terephthalate, polystyrene, and polypropylene, thin metal films, and multilayer films of two or more of them, because they are inexpensive, and they have satisfactory adhesion to the hydrophilic absorbent and have high processability.

The absorption portion is not limited, if it permits rapid absorption of the oral secreta liquid to be achieved in the internal hydrophilic absorbent exposed from the openings of the water resistant substrate.

The liquid holding portion is not limited, if it is continuous with the absorption portion, if it is covered with the water resistant substrate, and if it is kept from drying.

The held liquid amount confirmation/display portion is not limited, if it is located above the liquid holding portion, and if it realizes a structure and a function such that the oral secreta liquid, absorbed from the absorption portion, wets the display portion to change optical transmission, whereby a colored portion on the back side of the hydrophilic absorbent in the display portion can be visually confirmed from the front.

The recovery hole is not limited, if it is located at the end surface side of the liquid holding portion, if it realizes a structure and a function such that the liquid holding portion, which has absorbed the oral secreta liquid and swollen, is pressurized toward the recovery hole, whereby the oral secreta liquid is recovered through the recovery hole, and if it has a shape which enables spotting of the recovered sample to be performed with high efficiency.

The pressurizing implement is not limited, if, during pressurization, it does not contaminate the recovered oral secreta liquid and does not damage the sampling/recovery instrument.

### Embodiments

Embodiments of the present invention will be described concretely, but in no way limit the invention.

### Embodiment 1 Oral secreta liquid sampling/recovery instrument

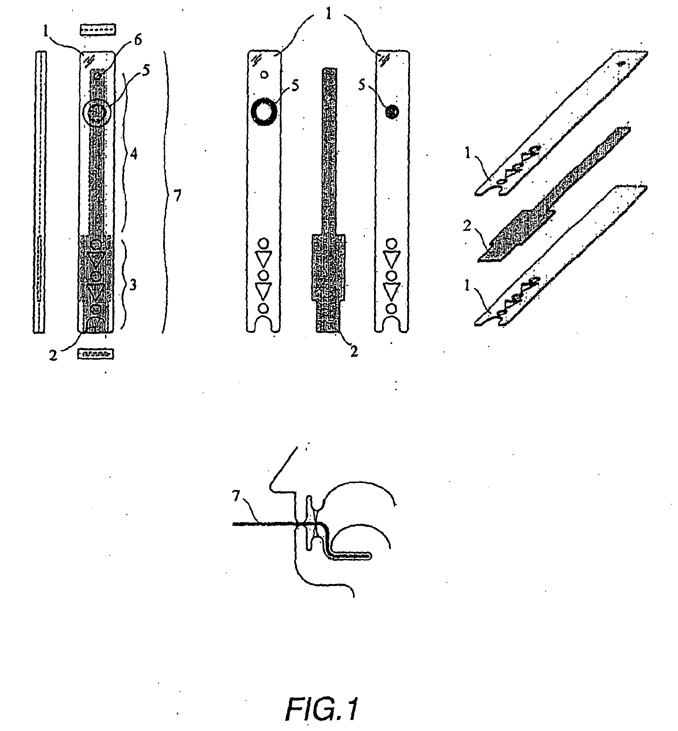

FIG. 1 shows an example of the oral secreta liquid sampling/recovery instrument in the present embodiment.

A hydrophilic absorbent 2 was disposed between two water resistant substrates 1 each consisting of a two-layer (nylon/polyethylene) film (manufacturer: Seisan Nihonsha), with the polyethylene side of the film being located internally. The hydrophilic absorbent 2 had been prepared by impregnating a nonwoven fabric (weight: 80 g/m²) (manufacturer: OMIKENSHI), which contained chitin-chitosan, an antibacterial ingredient, with 0.01 w/v% of a benzalkonium chloride solution (manufacturer: Nihon Seiyaku), a bactericidal ingredient, and then drying the impregnated nonwoven fabric. This combination was instantaneously heated at about 200°C, while being pressurized, whereby the water resistant substrates 1 and the hydrophilic absorbent 2 were thermocompression bonded to produce a sampling/recovery instrument 7.

In each of the water resistant substrates 1, the openings of the absorption portion 3 and the recovery hole 6 were formed beforehand, and the colored portion of the held liquid amount confirmation/display portion 5 was also created beforehand, by coloration, between the nylon and the polyethylene. This precluded contact with an user of a coloring ink and with an oral secreta liquid 8.

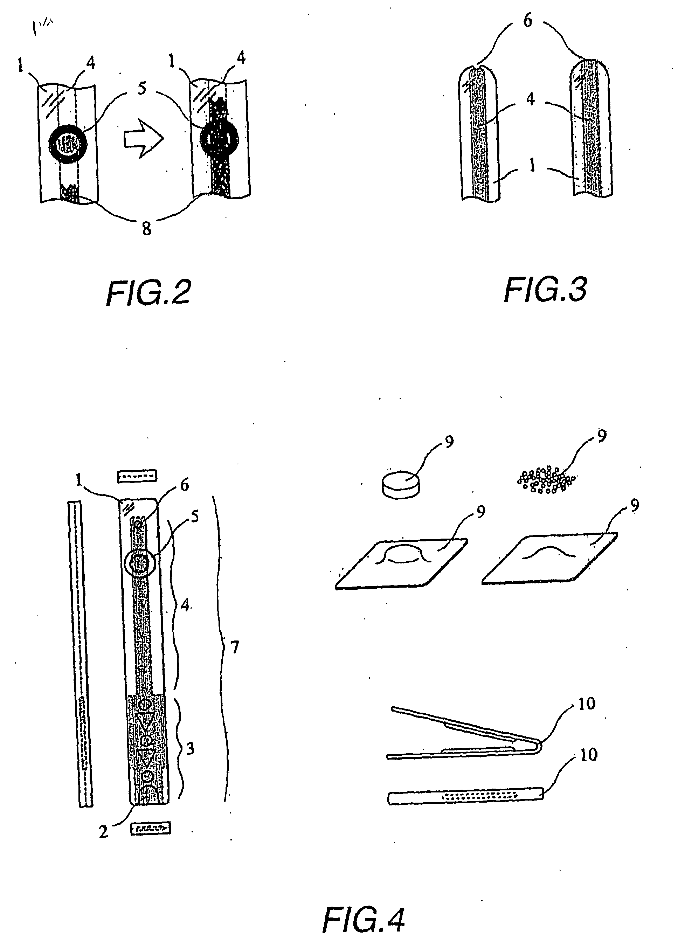

FIG. 2 shows the held liquid amount confirmation/display portion of the oral secreta liquid sampling/recovery instrument.

Upon contact with the absorption portion 3, the oral secreta liquid 8 showed satisfactory absorbability. The oral secreta liquid 8 contacted the bactericidal ingredient and antibacterial ingredient contained in the absorption portion 3, became absorbed to the liquid holding portion 4, and moved. When the oral secreta liquid 8 passed the held liquid amount confirmation/display portion 5, the confirmation/display portion 5 was wetted and changed in optical transmission, whereby a colored portion on the back side of the hydrophilic absorbent 2 could be visually confirmed from the front.

Thus, a required amount of the sample could be easily confirmed. The liquid holding portion 4 was pressurized toward the recovery hole 6, thereby enabling the oral secreta liquid 8 to be recovered through the recovery hole 6.

Particularly, the oral secreta liquid 8, recovered after sampling, had a viscous substance, which seemed to be mucin, trapped by the hydrophilic absorbent 2, and became serous. It was also confirmed that residues of foods, etc. were trapped by the hydrophilic absorbent 2 to some extent.

A material having adsorptivity may be added to the recovery hole 6. For example, a layer of nylon having protein adsorptivity by double bonds (manufacturer: Nihon Pall) may be provided. By so doing, binding of protein by the adsorption layer is noted, and impartment of a separating function is achieved. Furthermore, a filtration layer comprising polyether sulfone (manufacturer: Nihon Pall) may be provided, thereby making separation possible.

As described above, diverse functions can be imparted by providing a two-layer configuration including the hydrophilic absorbent 2, or a multilayer configuration including it.

FIG. 3 shows the recovery hole of the oral secreta liquid sampling/recovery instrument.

By changing the shape of the recovery hole 6 variously, it became possible to perform spotting of the recovered sample to POCT equipment with a high efficiency.

### Embodiment 2 Oral secreta liquid sampling/recovery kit

FIG. 4 shows an example of the configuration of an oral secreta liquid sampling/recovery kit in the present embodiment.

To promote salivation, there was concomitantly used a bactericide incorporating suitable additives not interfering with D-glucose determination, such as a flavor and an aromatic, in addition to the aforementioned bactericidal ingredient.

A troche (1,400 mg) containing 6.0 mg of cetylpyridinium chloride as a bactericidal ingredient was dissolved sublingually. As a result, a satisfactory amount of salivation was obtained.

If a bactericide 9 is to be used concomitantly, the amount of the bactericidal ingredient contained in the hydrophilic absorbent 2 of the sampling/recovery instrument 7 should be decreased.

By using a pressurizing implement 10 concurrently, the conditions for pressurization of the liquid holding portion 4 were increased in reproducibility.

In the above embodiments, the particular shapes were illustrated in connection with the oral secreta liquid sampling/recovery simplified instrument, but they are not limitative.

Similarly, the bactericide and the pressurizing implement are not restrictive.

## Claims

1. An oral secreta liquid sampling/recovery instrument, comprising:
a hydrophilic absorbent, which is a flexible nonwoven fabric containing, at least, a bactericidal ingredient and/or an antibacterial ingredient; and
a water resistant substrate, which is a transparent flexible film,
said hydrophilic absorbent having a structure in which a face and back thereof are entirely covered with said water resistant substrate by thermocompression bonding, with a partial opening portion being retained, and
wherein an absorption portion, a liquid holding portion, a held liquid amount confirmation/display portion, and a recovery hole for the oral secreta liquid are arranged consecutively in a straight line.

2. The oral secreta liquid sampling/recovery instrument according to claim 1, wherein said held liquid amount confirmation/display portion has a structure such that when the oral secreta liquid is absorbed to said hydrophilic absorbent, said hydrophilic absorbent is wetted and changed in optical transmission, whereby a colored portion on a back side of said hydrophilic absorbent can be visually confirmed from a front.

3. The oral secreta liquid sampling/recovery instrument according to claim 1 or 2, having a structure such that said liquid holding portion swollen upon absorption of the oral secreta liquid to said hydrophilic absorbent is pressurized toward said recovery hole, whereby the oral secreta liquid is recovered through said recovery hole.

4. The oral secreta liquid sampling/recovery instrument according to any one of claims 1 to 3, wherein said bactericidal ingredient and said antibacterial ingredient have a function of sterilizing and counteracting oral bacteria decomposing, at least, D-glucose.

5. The oral secreta liquid sampling/recovery instrument according to any one of claims 1 to 4, wherein said bactericidal ingredient and said antibacterial ingredient consist of a single component or a plurality of components, said bactericidal ingredient is cetylpyridinium chloride, benzalkonium chloride, or benzethonium chloride among quaternary ammonium salts, and said antibacterial ingredient is chitin-chitosan, silver, copper, or zinc.

6. An oral secreta liquid sampling/recovery kit, comprising the oral secreta liquid sampling/recovery instrument according to any one of claims 1 to 5, a bactericide, and a tweezers-shaped pressurizing implement.

7. The oral secreta liquid sampling/recovery kit according to claim 6, wherein said bactericide is in a form of a gas or a solid which does not dilute the oral secreta liquid, and is in a dosage form which is an aerosol, a capsule, granules, a powder, a tablet, a troche, or pills.

8. An oral secreta liquid sampling/recovery method using the sampling/recovery instrument or the sampling/recovery kit according to any one of claims 1 to 7.
